# EUROPEAN PATENT APPLICATION

(11) **EP 1 319 643 A2**
(43) Date of publication of application: **18.06.2003**
(21) Application number: 02027065.8
(22) Date of filing: 03.12.2002
(51) Int. Cl.: C07B 63/04, C07D 401/14

(54) **Macromolecular HALS with a definite molecular weight**

(30) Priority: 11.12.2001 IT MI20012598
(71) Applicant: 3V SIGMA S.p.A, 20121 Milano (IT)
(72) Inventor: Raspanti, Giuseppe, 24100 Bregamo BG (IT); Zanchi, Giorgio, 24100 Bregamo BG (IT)
(74) Representative: Minoja, Fabrizio, Dr.

(57) **Abstract**

The present invention relates to macromolecular derivatives with a definite molecular weight, containing a 2,2,6,6-tetramethyl-4-piperidyl group and to their use as light, heat and oxidation stabilizers for synthetic polymers and other organic materials, and to the materials thus stabilized.

## Description

### Background of the invention

Derivatives containing the 2,2,6,6-tetramethyl-4-piperidyl group, in the following denoted by the acronym HALS (Hindered Amines Light Stabilizers) and their use as light stabilizers for the synthetic polymers are well known in the technical literature. They include both monomeric molecules, with a relatively low molecular weight, and oligomeric or polymeric molecules with a higher average molecular weight. The latter products are practically mixtures of different molecular weights.

Examples of monomeric molecules are disclosed in US 4,530,950, DOS 1,929,928, US 3,640,928, US 3,640,928; examples of oligomeric or polymeric molecules are the ones described in US 4,477,615, US 4,233,412, US 4,331,586, DOS 2,636,144, DOS 2,456,864, US 4,315,859, US 4,104,248, US 4,086,204, US 4,038,280.

The choice of the product to be used for obtaining the highest stability of a material depends on the kind of the material itself, the conditions which it is subjected to during use, the length of use and the features which are intended to be particularly protected.

These features are first of all the appearance and the mechanical strength.

According to the technical literature (see: Research Disclosure n° 20936, published on September 1981 by F. Gucumus, Ciba-Geigy; Proceedings of "14ème Journées d'Etudes sur le Vieillissemente des Polimères" - Bandol, September 22, 2000) a correlation exists between the molecular weight and the performance. Generally speaking, low molecular weights have a minor resistance to migration and extraction in comparison with higher molecular weights; on the other hand, a too high molecular weights can be less effective in the stabilization.

### Summary of the invention

The invention relates to macromolecular HALS, with molecular weights not polydispersed, well defined and variable within a wide range, obtainable by suitably selecting the intermediates. The invention also concerns the process for the preparation thereof. By this means, a number of stabilizers are available, between which the most suitable to a specific need of use can be chosen.

### Detailed description of the invention

More particularly, the invention relates to compounds of formula (I) 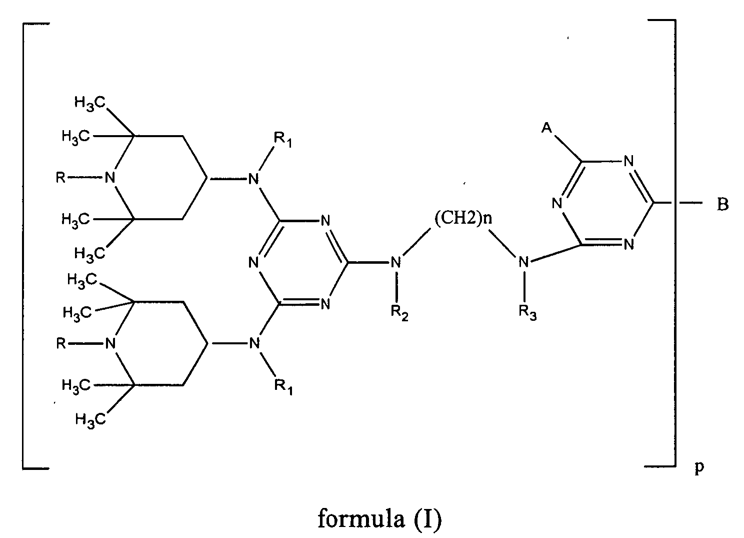 in which R is H, methyl or an-O-R4 group; R4 is C1-C8 linear or branched alkyl or cyclohexyl; R1 is H or C1-C8 linear or branched alkyl; R2 and R3, which can be the same or different, are H or C1-C8 linear or branched alkyl or a group of formula (II) 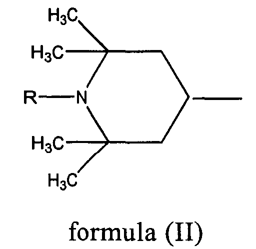 A is a group -N-R2R3 or a group of formula (III) 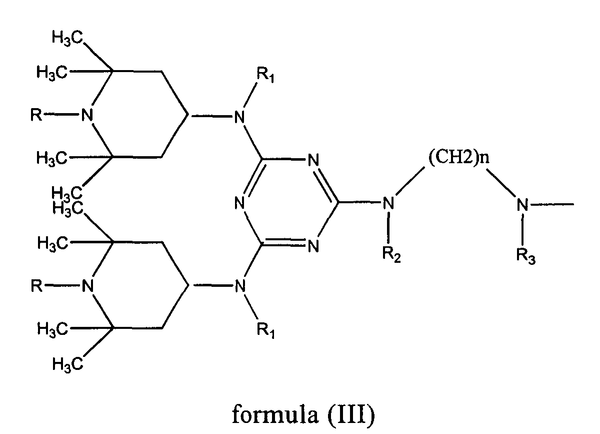 in which R, R1, R2, R3 have the meanings previously defined;
n is 2 to 6;
p is 2 to 5;
B is a group of formulas (IV) to (VIII): 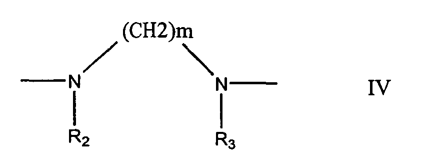 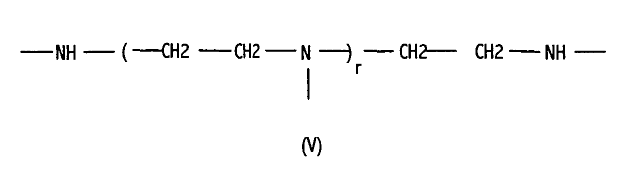 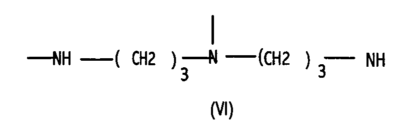 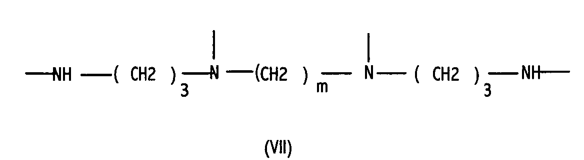 in which m is 2 to 12 and r is 1 to 3 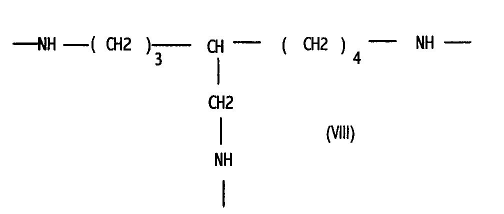 R is preferably H or methyl or the group OR4 where R4 is as previously defined, preferably cyclohexyl.

R1 is preferably H, methyl or butyl.

R2 and R2 preferably are H or a group of formula (II).

A is preferably a group of formula (II) or (III).

The preferred compounds of the present invention are those in which R1 is butyl, R2 and R3 are hydrogen, p has the values 2 to 4 and n is 6.

The compounds of the present invention are characterised by well defined, not polydispersed molecular weights.

The present invention also relates to a process for the preparation of compounds (I), which process consists of the following steps:
a compound of formula (IX): 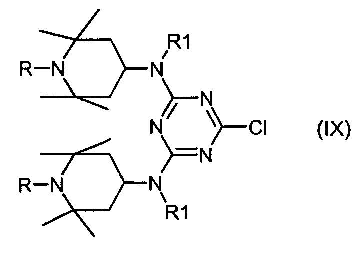 is reacted with the required amount of an amine of formula (X): 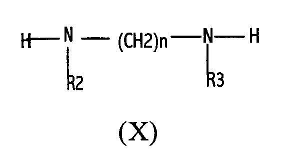 to give a compound of formula (XI):

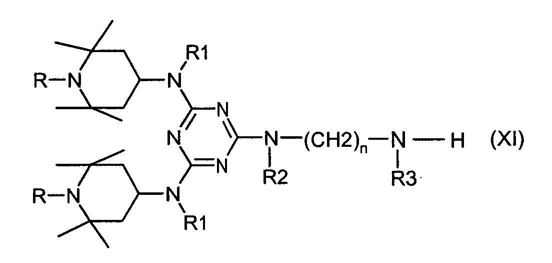

Two moles of the compound of formula (XI) are further reacted with one mole of cyanuric chloride to obtain the intermediate of formula (XII): 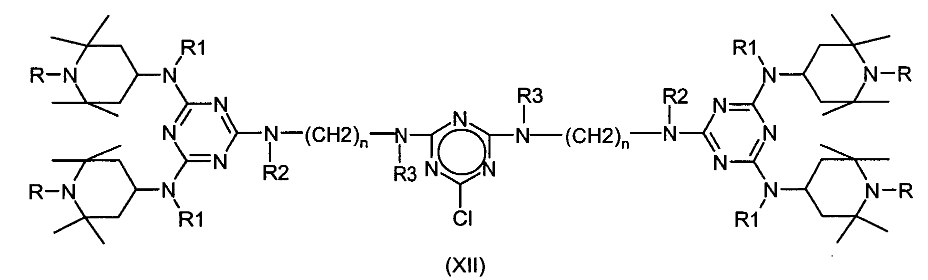 which is finally reacted with a stoichiometric amount of the polyamines comprising the groups shown in the formulas from (IV) to (VIII) thus obtaining the products of the present invention.

An alternative process for the preparation of compounds of formula (I) comprises the reaction of the compounds of formula (XI) with the compounds of formula (XIII): 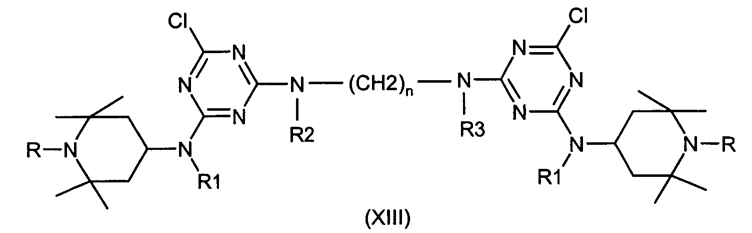 in which R, R1, R2, R3 have the meanings previously mentioned.

In the formulas (X), (XI), (XII) and (XIII), the methyl groups in the positions 2 and 6 of the piperidyl ring are represented as

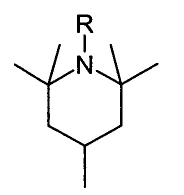

The compounds of formula (I) in which R = -O-R4 can be prepared from the corresponding compounds in which R = H by reaction with aliphatic or cycloaliphatic hydrocarbon in presence of peroxides or hydroperoxides and molybdenum oxide.

The resulting compounds have well defined, not polydispersed molecular weight, and consist of a single oligomer, instead of a mixture of many of them.

The compounds of formula (I), alone or in mixture with other compounds of formula (I), are very effective in improving the light, heat and oxidation stabilization of organic materials, especially synthetic polymers and copolymers. Polypropylene and polyethylene in the form of films, fibers or also thick objects, are preferred polymers. The compounds wherein R is an -OR4 group are particularly suited for agricultural films, as this group is more resistant than hydrogen and alkyl residues against the deactivating action by pesticides, especially sulphur-containing pesticides, and by acids.

When the R- residue in the formula (I) is the-O-R4 group, the compounds of the invention can also be used as flame retardants.

Examples of materials that can be stabilized are:
a) Polymers of monoolefins and di-olefins, their co-polymers and mixtures of homo and co-polymers of monoolefins and di-olefins. Examples of monoolefins and di-olefins polymers are polymers of propylene, ethylene, isobutylene, methylpentene, isoprene, butadiene, cycloolefins, such as cyclopentene and norbornene. The polymers can be crosslinked and have different molecular weights, for example high density polyethylene (HDPE), high density and high molecular weight polyethylene (HDPE-HMW), high density and ultrahigh molecular weight polyethylene (HDPE-UHMW), medium density polyethylene (MDPE), low density polyethylene (LDPE), linear low density polyethylene (LLDPE).
b) Polystyrene, poly(p-methylstyrene), poly(α -methylstyrene) and copolymers of styrene or methylstyrene with dienes or acrylic monomers, for example styrene/butadiene, styrene/acrylonitrile, styrene/alkyl acrylate, styrene/alkyl methacrylate, styrene/maleic anhydride, styrene/butadiene/acrylonitrile or mixture of these polymers and copolymers; polymers of styrene or methylstyrene or acrylic monomers grafted on polybutadiene or polybutadiene/styrene or polybutadiene/acrylonitrile or ethylene/propylene/diene terpolymers or mixture of copolymers known as ABS, MBS, AES.
c) Halogen-containing polymers such as polyvinyl chloride or fluoride, polyvinylidene chloride or fluoride, as well as their copolymers such as vinyl chloride/vinylidene chloride, vinyl chloride/vinyl acetate; chlorinated rubbers, polychloroprene, chlorinated and brominated copolymers of isobutylene/isoprene, chlorinated polyethylene, copolymers of ethylene and chlorinated ethylene, epichlorohydrin copolymers.
d) Acrylic polymers such as polyacrylates, polymethylmethacrylates, polyacrylamides, polyacrylonitrile, butyl acrylate impact-modified acrylic polymers.
e) Vinyl polymers such as polyvinyl alcohol, polyvinyl acetate, vinyl alcohol/vinyl acetate copolymers, polyvinyl esters, polyvinyl butyral.
f) Homo and co-polymers of cyclic ethers such as polyalkylene glycols, polymers and copolymers of ethylene oxide, propylene oxide; polyacetals such as polyoxymethylene and polyacetals modified with thermoplastic polyuretanes or acrylates.
g) Polyurethanes obtained from hydroxyl terminated polyethers or polyesters with aliphatic or aromatic isocyanates or polyisocyanates.
h) Polyamides derived from aliphatic or aromatic diamines with aliphatic or aromatic dicarboxylic acids or derived from amino-carboxylic acids, for example polyamide 4, polyamide 6, polyamide 6/6, polyamide 11, 12; block copolymers of the aforementioned polyamides with polyolefins or polyethers.
i) Polyureas, polyimides, polyamide-imides, polyetherimids, polyesterimids, polyhydantoins and polybenzimidazoles.
j) Polyesters derived from dicarboxylic acids and diols or from hydroxycarboxylic acids, for instance polyethylene terephthalate, polybutylene terephthalate; block copolymers obtained from hydroxyl-terminated polyethers with dicarboxylic acids.
k) Polycarbonates and polyester carbonates.
l) Polysulfones and copolymers containing sulfonic groups.
m) Phenol/formaldheyde resins, urea/formaldheyde resins and melamine/formaldheyde resins.
n) Drying alkyd resins, unsaturated polyesters resins, pre-polymers for drying or polymerisable coatings.
o) Epoxy resins derived from aliphatic, cycloaliphatic, heterocyclic or aromatic glycidyl compounds, for example diglycidyl ether of bisphenol A, crosslinked with customary hardeners such as anhydrides or amines.
p) Natural polymers such as cellulose, rubber, gelatin and chemically modified derivatives thereof such as cellulose acetates, methyl cellulose, carboxymethyl cellulose.
q) Blends of the polymers mentioned from a) to p).
r) Natural and synthetic latices, water in oil and oil in water emulsions, mineral and vegetable oils, waxes and fats, optionally synthetically modified also.

For the purposes of the present invention, the compounds of formula (I) can also be used in mixture with other stabilizers based upon hindered amines, for example those disclosed in US 4,477,615, DOS 2,456,864, DOS 2,636,144, DOS 1,929,928.

The invention further relates to compositions comprising organic materials susceptible to degradation induced by light, heat or oxidation containing at least a compound of formula (I). The organic material is preferably a synthetic polymer, more particularly a polyolefin such as polyethylene or polypropylene.

The amount of compounds of formula (I) or the mixture thereof to be used is from 0.01% to 5%, preferably from 0.05% to 2%, more preferably from 0.05% to 1%, referred to the weight of the material to be stabilized. The amount depends anyway on the nature of the material and on the effect to be obtained.

The compounds of formula (I) can be added to the finished polymeric materials or during their manufacturing and can be incorporated in the pure form or in mixture with other additives suitable for the intended scope, as for instance antioxidants, UV absorbers, heat stabilizers, such as the ones listed below. Furthermore, they can be in form of powder, granules or pellets, encapsulated in waxes, oils or polymers.

If required, the materials to be stabilized can already contain, or can be subsequently added with, other conventional additives as antioxidants, UV absorbers, fillers, nickel stabilizers, plasticizers, every kind of anti-aging stabilizers, corrosion inhibitors and metal deactivators such as the ones listed below.

Examples of the aforementioned conventional additives are:
1. Antioxidants
   1.1. Alkylated phenols, for example 2,6-di-tert-butyl-4-methylphenol; 2-tert-butyl-4,6-dimethylphenol; 2,6-di-tert-butyl-4-ethylphenol; 2,6-di-tert-butyl-4-n-butylphenol; 2,6-di-tert-butyl-4-isobutylphenol; 2,6-dicyclopentyl-4-rnethylphenol; 2-(α-methylcyclohexyl)-4,6-dimethyl-phenol; 2,6-dioctadecyl-4-methylphenol; 2,4,6-tricyclohexyl-phenol; 2,6-di-tert-butyl-4-methoxymethyl-phenol; nonylphenols which are linear or branched in the side chains, for example, 2,6-di-nonyl-4-methylphenol; 2,4-dimethyl-6-(1'-methylundecyl)phenol; 2,4-dimethyl-6-(1'-methylheptadecyl)phenol; and mixtures thereof.
   1.2. Alkylthiomethylphenols, for example 2,4-dioctylthiomethyl-6-tert-butylphenol; 2,4-dioctylthiomethyl-6-methylphenol; 2,4-dioctylthio-methyl-6-ethylphenol; 2,6-di-dodecylthiomethyl-4-nonylphenol.
   1.3. Hydroquinones and alkylated hydroquinones, for example 2,6-di-tert-butyl-4-methoxyphenol; 2,5-di-tert-butylhydroquinone; 2,5-di-tert-amylhydroquinone; 2,6-diphenyl-4-octadecyloxyphenol; 2,6-di-tert-butylhydroquinone; 2,5-di-tert-butyl-4-hydroxyanisole; 3,5-di-tert-butyl-4-hydroxyanisole; 3,5-di-tert-butyl-4-hydroxyphenyl stearate; bis-(3,5-di-tert-butyl-4hydroxyphenyl) adipate.
   1.4. Tocopherols, for example α-tocopherol, β-tocopherol, γ-tocopherol, δ-tocopherol and mixtures thereof (Vitamin E).
   1.5. Hydroxylated thiodiphenyl ethers, for example 2,2'-thiobis-(6-tert-butyl-4-methylphenol); 2,2'-thiobis(4-octylphenol); 4,4-thiobis-(6-tert-butyl-3-methylphenol); 4,4'-thiobis-(6-tert-butyl-2-methylphenol); 4,4'-bis-(2,6-dimethyl-4-hydroxyphenyl)disulfide.
   1.6. Alkylidenebisphenols, for example 2,2'-methylenebis(6-tert-butyl-4-methylphenol); 2,2'methylenebis(6-tert-butyl-4-ethylphenol); 2,2'-methylenebis[4-methyl-6-(α-methylcyclohexyl)-phenol]; 2,2'- methylene bis-(4-methyl-6-cyclohexylphenol); 2,2'-methylenebis(6-nonyl-4-methyl-phenol); 2,2'-methylenebis-(4,6-di-tert-butylphenol); 2,2'-ethylidenebis-(4,6-di-tert-butyl-phenol); 2,2'-ethylidenebis-(6-tert-butyl -4-isobutylphenol); 2,2'-methylenebis[6-(α-methylbenzyl)-4-nonylphenol]; 2,2'-methylene-bis[6-(α,α-dimethylbenzyl)-4-nonylphenol]; 4,4'-methylenebis(2,6-di-tert-butylphenol); 4,4'-methylenebis-(6-tert-butyl-2-methylphenol); 1,1-bis(5-tert-butyl-4-hydroxy-2-methylphenyl)-butane; 2,6-bis(3-tert-butyl-5-methyl-2-hydroxybenzyl)-4-methylphenol;1,1,3-tris(5-tert-butyl-4-hydroxy-2-methyl-phenyl)butane; 1,1-bis(5-tert-butyl-4-hydroxy-2-methyl-phenyl)-3-n-dodecyl-mercaptobutane; ethylene glycol bis[3,3-bis(3'-tert-butyl-4'-hydroxyphenyl)butyrate]; bis(3-tert-butyl-4-hydroxy-5-methyl- phenyl)-dicyclopentadiene; bis-[2-(3'-tert-butyl-2'-hydroxy-5 '-methylbenzyl)-6-tert-butyl-4-methylphenyl]terephthalate; 1,1-bis-(3,5-dimethyl-2-hydroxyphenyl)-butane; 2,2-bis-(3,5-di-tert-butyl-4-hydroxyphenyl)-propane; 2,2-bis-(5-tert-butyl-4-hydroxy-2-methylphenyl)-4-n-dodecylmercaptobutane; 1,1,5,5-tetra-(5-tert-butyl-4-hydroxy-2-methylphenyl)-pentane.
   1.7. 0-, N- and S-benzyl compounds, for example 3,5,3',5'-tetra-tert-butyl-4,4'-dihydroxy-di-benzyl ether, octadecyl-4-hydroxy-3,5-dimethylbenzylmercaptoacetate, tridecyl-4-hydroxy-3,5-di-tert-butylbenzylmercaptoacetate, tris(3,5-di-tert-butyl-4-hydroxybenzyl)amine, bis(4-tert-butyl-3-hydroxy-2,6-dimethylbenzyl)dithioterephthalate, bis(3,5-di-tert-butyl-4-hydroxy-benzyl)sulfide, isooctyl-3,5-di-tert-butyl-4-hydroxybenzylmercaptoacetate.
   1.8. Hydroxybenzylated malonates, for example dioctadecyl-2,2-bis-(3,5-di-tert-butyl-2-hydroxybenzyl)-malonate, di-octadecyl-2-(3-tert-butyl-4-hydroxy-5-methylbenzyl)-malonate, didodecylmercaptoethyl-2,2-bis-(3,5-di-tert-butyl-4-hydroxybenzyl)-malonate, bis-[4-(1,1,3,3-tetramethylbutyl) phenyl]-2,2-bis(3,5-di-tert-butyl-4-hydroxybenzyl) malonate.
   1.9. Aromatic hydroxybenzyl compounds, for example 1,3,5-tris-(3,5-di-tert-butyl-4-hydroxy-benzyl)-2,4,6-trimethylbenzene, 1,4-bis(3,5-di-tert-butyl-4-hydroxybenzyl)-2,3,5,6-tetramethylbenzene, 2,4,6-tris-(3,5-di-tertbutyl-4-hydroxybenzyl)phenol.
   1.10. Triazine Compounds, for example 2,4-bis-(octylmercapto)-6-(3,5-di-tert-butyl-4-hydroxyanilino)-1,3,5-triazine, 2-octylmercapto-4,6-bis-(3,5-di-tert-butyl-4-hydroxyanilino)-1,3,5-triazine, 2-octylmercapto-4,6-bis-(3,5-di-tert-butyl-4-hydroxyphenoxy)-1,3,5-triazine, 2,4,6-tris-(3,5-di-tert-butyl-4-hydroxyphenoxy)-1,2,3-triazine, 1,3,5-tris-(3,5-di-tert-butyl-4-hydroxybenzyl)-isocyanurate, 1,3,5-tris(4-tert-butyl-3-hydroxy-2,6-dimethyl-benzyl)-isocyanurate, 2,4,6-tris- (3,5-di-tert-butyl-4-hydroxy-phenylethyl)-1,3,5-triazine, 1,3,5-tris-(3,5-di-tert-butyl-4hydroxyphenyl-propionyl)-hexahydro-1,3,5-triazine, 1,3,5-tris-(3,5-dicyclohexyl-4-hydroxybenzyl) isocyanurate.
   1.11.Benzylphosphonates, for example dimethyl-2,5-di-tert-butyl-4-hydroxybenzylphosphonate, diethyl-3,5-di-tert-butyl-4-hydroxybenzylphosphonate, dioctadecyl-3,5-di-tert-butyl-4-hydroxybenzylphosphonate, dioctadecyl-5-tert-butyl-4-hydroxy-3-methylbenzylphosphonate, 3,5-di-tert-butyl-4-hydroxybenzylphosphonic acid monoethyl ester calcium salt.
   1.12. Acylaminophenols, for example 4-hydroxylauranilide, 4-hydroxystearanilide, octyl-N-(3,5-di-tert-butyl-4-hydroxyphenyl)carbamate.
   1.13. Esters of β-(3,5-di-tert-butyl-4-hydroxyphenyl)propionic acid with mono- or polyhydric alcohols, such as methanol, ethanol, n-octanol, i-octanol, octadecanol, 1,6-hexanediol, 1,9-nonanediol, ethylene glycol, 1,2-propanediol, neopentyl glycol, thiodiethylene glycol, diethylene glycol, triethylene glycol, pentaerythritol, tris(hydroxyethyl) isocyanurate, N,N'-bis(hydroxyethyl)oxamide, 3-thiaundecanol, 3-thiapentadecanol, trimethylhexanediol, trimethylolpropane, 4-hydroxymethyl-1-phospha-2,6,7-trioxabicyclo[2.2.2]octane.
   1.14. Esters of β-(5-tert-butyl-4-hydroxy-3-methylphenyl)propionic acid with mono- or polyhydric alcohols, such as methanol, ethanol, n-octanol, i-octanol, octadecanol, 1,6-hexanediol, 1,9-nonanediol, ethylene glycol, 1,2-propanediol, neopentyl glycol, thiodiethylene glycol, diethyIene glycol, triethylene glycol, pentaerythritol, tris-(hydroxyethyl) isocyanurate, N,N'-bis-(hydroxyethyl)oxamide, 3-thiaundecanol, 3-thiapentadecanol, trimethylhexanediol, trimethylolpropane, 4-hydroxymethyl-1-phospha-2,6,7-trioxabicyclo[2.2.2]octane.
   1.15. Esters of β-(3-,5-dicyclohexyl-4-hydroxyphenyl)propioníc acid with mono- or polyhydric alcohols, such as methanol, ethanol, octanol, octadecanol, 1,6-hexanediol, 1,9-nonanediol, ethylene glycol, 1,2-propanediol, neopentyl glycol, thiodiethylene glycol, diethylene glycol, triethylene glycol, pentaerythritol, tris(hydroxyethl)isocyanurate, N,N'-bis(hydroxyethyl)-oxamide, 3-thiaundecanol, 3-thiapentadecanol, trimethylhexanediol, trimethylolpropane, 4-hydroxymethyl-1-phospha-2,6,7-trioxabicyclo[2.2.2]-octane.
   1.16. Esters of 3,5-di-tert-butyl-4-hydroxyphenyl acetic acid with mono- or polyhydric alcohols, such as methanol, ethanol, octanol, octadecanol, 1,6-hexanediol, 1,9-nonanediol, ethylene glycol, 1,2-propanediol, neopentyl glycol, thiodiethylene glycol, diethylene glycol, triethylene glycol, pentaerythritol, tris(hydroxyethyl)isocyanurate, N,N'-bis(hydroxyethyl)-oxamide, 3-thiaundecanol, 3-thiapentadecanol, trimethylhexanediol, trimethylolpropane, 4-hydroxymethyl-1-phospha-2,6,7-trioxabicyclo[2.2.2]-octane.
   1.17. Amides of β-(3,5-di-tert-butyl-4-hydroxyphenyl)propíonic acid e.g. N,N'-bis-(3,5-di-tert-butyl-4-hydroxyphenylpropionyl)-hexamethylenediamide, N,N'-bis-(3,5-di-tert-butyl-4-hydroxy-phenylpropionyl)-trimethylenediamide, N,N'-bis-(3,5-di-tert-butyl-4-hydroxyphenylpropionyl)hydrazide, N,N'-bis(2-(3-[3,5-di-tert-butyl-4-ydroxyphenyl]propionyloxy)ethyl]oxamide.
   1.18. Ascorbic acid (vitamin C)
   1.19. Aminic antioxidants, such as N,N'-di-isopropyl-p-phenylenedíamine, N,N'-di-sec-butyl-p-phenylenedamine, N,N'-bis(1,4-dimethylpentyl)-p-phenylenediamine, N,N'-bis(1-ethyl-3-methylpentyl)-p-phenylenediamine, N,N'-bis(1 -methylheptyl)-p-phenylenediamine, N,N'-dicyclohexyl-p-phenylenediamine, N,N'-diphenyl-p-phenylenediamine, N,N'-bis(2-naphthyl)-p-phenylenediamine, N-isopropyl-N'-phenyl-p-phenylenediamine, N-(1,3-dimethyIbutyl)-N'-phenyl-p-phenylenediamine, N-(1-methylheptyl)-N'-phenyl-p-phenylenediamine, N-cyclohexyl-N'-phenyl-p-phenylenediamine, 4-(p-toluenesulfamoyl)diphenylamine, N,N'-dimethyl--N,N'-di-sec-butyl-p-phenylenediamine, diphenylamine, N-allyldiphenylamine, 4-isopropoxydiphenylamine, N-phenyl-1-naphthylamine, N-(4-tert-octylphenyl)-1-naphthylamine, N-phenyl-2-naphthylamine, p,p'-di-tert-octyldiphenylamine, 4-n-butylaminophenol, 4-butyrylaminophenol, 4-nonanoylaminophenol, 4-dodecanoylaminophenol, 4-octadecanoylaminophenol, bis-(4-methoxyphenyl)-amine, 2,6-di-tert-butyl-4-dimethylaminomethyl-phenol, 2,4'-damino-diphenylmethane, 4,4-diamino-diphenyl-methane, N,N,N',N'-tetramethyl-4,4'-diaminodiphenylmethane, 1,2-bis[(2-methylphenyl)amino]ethane, 1,2-bis(phenylamino)propane, (o-tolyl)biguanide, bis[4-(1',3'-dimethylbutyl)phenyl]amine, tertoctylated N-phenyl-1-naphthylamine, a mixture of mono- and dialkylated tert-butyl/tert-octyldiphenylamines, a mixture of mono- and dialkylated nonyldiphenylamines, a mixture of mono- and dialkylated dodecyldiphenylamines, a mixture of mono- and dialkylated isopropyl/isohexyldiphenylamines, a mixture of mono- and dialkylated tert-butyldiphenylamines, 2,3-dihydro-3,3-dimethyi-4H-1,4-benzothiazine, phenothiazine, a mixture of mono- and dialkylated tert-butyl/tert-octylphenothiazines, a mixture of mono- and dialkylated tert-octyl-phenothiazines, N-allylphenothiazine, N,N,N',N'-tetraphenyl-1,4-diaminobut-2-ene, N,N-bis-(2,2,6,6-tetramethyl-piperid-4-yl-hexamethlienediamine, bis-(2,2,6,6-tetramethylpiperid-4-yl)sebacate, 2,2,6,6-tetramethylpiperidin-4-one, 2,2,6,6-tetramethyl piperidin-4-ol.
2. UV absorbers and light stabilisers
   2.1. 2-(2.'-Hydroxyphenyl)benzotriazoles, such as 2-(2'-hydroxy-5'-methylphenyl)-benzo-triazole, 2-(3',5'-di-tert-butyl-2'-hydroxyphenyl)-benzotriazole, 2-(5'-tert-butyl-2'-hydroxyphenyl) benzotriazole, 2-(2'-hydroxy-5'-(1,1,3,3-tetramethylbutyl)phenyl)benzotriazole, 2-(3',5'-dí tert-butyl-2'-hydroxyphenyl)-5-chloro-benzotriazole, 2-(3'-tert-butyl-2'-hydroxy-5'-methylphenyl)-5-chloro-benzotriazole, 2-(3'-sec-butyl-5'-tert-butyl-2'-hydroxyphenyl)benzotriazole, 2-(2' hydroxy-4'-octyloxyphenyl)benzotriazole, 2-(3',5'-di-tert-amyl-2'-hydroxyphenyl)-benzotriazole, 2-(3',5'-bis-(α,α-dimethyl-benzyl)-2'-hydroxy-phenyl)-benzotriazole, 2-(3'-tert-butyl-2-hydroxy-5'-(2- octyloxycarbonyl-ethyl) phenyl)-5-chloro-benzotriazole, 2-(3'-tert-butyl-5'-[2-(2-ethylhexyloxy)-carbonylethyl]-2'-hydroxyphenyl)-5-chloro-benzotriazole, 2-(3'-tert-butyl-2'-hydroxy-5'-(2-methoxycarbonyl)-ethyl)phenyl)-5-chloro-benzotriazole, 2-(3'-tert-butyl-2'-hydroxy 5'-(2-methoxy-carbonyl-ethyl)phenyl benzotriazole, 2-(3'-tert-butyl-2'hydroxy-5'-(2-octyloxy-carbonyl-ethyl)phenyl)benzotriazole, 2-(3'-tert-butyl-5'-[2-(2-ethylhexyloxy)carbonylethyl]-2'-hydroxy-phenyl)benzotriazole, 2-(3'-dodecyl-2'-hydroxy-5'-methylphenyl)benzotriazole, 2-(3'tert-butyl-2'-hydroxy-5'-(2-iso-octyloxy-carbonyl-ethyl)-phenylbenzotriazole, 2,2'-methylene-bis [4-(1,1,3,3-tetramethylbutyl)-6-benzotriazole-2-yl-phenol]; the transesterification product of 2-[3'-tert-butyl-5'-(2-methoxycarbonylethyl)-2'-hydroxyphenyl]-2H-benzotriazole with polyethylene glycol 300; [R-CH2CH2-COO-CH2CH2 -]₂- where R = 3'-tert-butyl-4'-hydroxy-5'-2H-benzotriazol-2-yl-phenyl, 2-[2'-hydroxy-3'-(α,α-dimethyl-benzyl)-5'-(1,1,3,3-tetramethylbutyl)-phenyl] benzotriazole; 2-[2'-hydroxy-3'-(1,1,3,3-tetramethyl-butyl)-5'-(α,α - dimethyl-benzyl)-phenyl]-benzotriazole.
   2.2. 2-Hydroxybenzophenones, such as 4-hydroxy, 4-methoxy, 4-octyloxy, 4-decyloxy, 4-dodecyloxy, 4-benzyloxy, 4,2,4'-trihydroxy and 2-hydroxy-4,4'-dimethoxy derivatives.
   2.3. Esters of substituted and unsubstìtuted benzoic acids, such as 4-tert-butyl-phenyl salicylate, phenyl salicylate, octylphenyl salicylate, díbenzoyl resorcinol, bis(4-tert-butylbenzoyl) resorcinol, benzoyl resorcinol, 2,4-di-tert-butylphenyl 3,5-di-tert-butyl-4-hydroxybenzoate, hexadecyl 3,5-di-tert-butyl-4-hydroxybenzoate, octadecyl 3,5-di-tert-butyl-4-hydroxybenzoate, 2-methyl-4,6-di-tert-butylphenyl 3,5-di-tert-butyl-4-hydroxybenzoate.
   2.4. Acrylates, such as ethyl (α-cyano-β,β-diphenylacrylate, isooctyl α-cyano-β,β-diphenylacrylate, methyl α-carbomethoxycinnamate, methyl-α-cyano-β-methyl-p-methoxy-cinnamate, butyl α-cyano-β-methyl-p-methoxycinnamate, methyl-α-carbomethoxy-p-methoxycinnamate and N-(β-carbomethoxy-β-cyanovinyl)-2-methylindoline.
   2.5. Nickel compounds, for example nickel complexes of 2,2'-thio-bis-[4-(1,1,3,3-tetra-methylbutyl)phenol], such as the 1:1 or 1:2 complex, with or without additional ligands such as n-butylamine, triethanolamine or N-cyclohexyldiethanolamine, nickel dibutyldithiocarbamate, nickel salts of monoalkyl esters, e.g. the methyl or ethyl ester, of 4-hydroxy-3,5-di-tert-butylbenzylphosphonic acid, nickel complexes of ketoximes, e.g. of 2-hydroxy-4-methylphenyl undecylketoxime, nickel complexes of 1-phenyl-4-lauroyl-5-hydroxypyrazole, with or without additional ligands.
   2.6. Sterically hindered amines, such as bis (2,2,6,6-tetramethyl-4-piperidyl)sebacate, bis(2,2,6,6-tetramethyl-4-piperidyl)succinate, bis(1,2,2,6,6-pentamethyl-4-piperidyl)sebacate, bis(1-octyloxy-2,2,6,6-tetramethyl-4-piperidyl)sebacate, bis(1,2,2,6,6-pentamethyl-4-piperidyl)-n-butyl-3,5-di-tert-butyl-4-hydroxybenzylmalonate, the condensate of 1-(2-hydroxyethyl)-2,2,6,6-tetramethyl-4-hydroxypiperidine and succinic acid, linear or cyclic condensates of N,N'-bis(2,2,6,6-tetramethyl-4-piperidyl)hexamethylenediamine and 4-tert-octylamino-2,6-dichloro-1,3,5-triazine, tris(2,2,6,6-tetramethyl-4-piperidyl) nitrilotriacetate, tetrakis (2,2,6,6-tetramethyl-4-piperidyl)-1,2,3,4-butane-tetracarboxylate, 1,1'-(1,2-ethanediyl)-bis (3,3,5,5-tetramethylpiperazinone), 4-benzoyl-2,2,6,6-tetramethylpiperidine, 4-stearyloxy-2,2,6,6-tetramethylpiperidine, bis-(1,2,2,6,6-pentamethylpiperidyl)-2-n-butyl-2-(2-hydroxy-3,5-di-tert-butyl-benzyl)malonate, 3-n-octyl-7,7,9,9-tetramethyl-1,3,8-triazaspiro-[4.5]decan-2,4-dione, bis(1-octyloxy-2,2,6,6-tetramethyl-piperidyl)sebacate, bis(1-octyloxy-2,2,6,6-tetramethylpiperidyl)-succinate, linear or cyclic condensates of N,N'-bis-(2,2,6,6-tetramethyl-4-piperidyl)hexamethylenediamine and 4-morpholino-2,6-dichioro-1,3,5-triazine, the condensate of 2-chloro-4,6-bis(4-n-butylamino-2,2,6,6-tetramethylpiperidyl)-1,3,5-triazine and 1,2-bis (3-aminopropylamino)ethane, the condensate of 2-chloro-4,6-di-(4-n-butylamino-1,2,2,6,6-pentamethylpiperidyl)-1,3,5-triazine and 1,2-bis-(3-aminopropylamino)ethane, 8-acetyl-3-dodecyl-7,7,9,9-tetramethyl-1,3,8-triazaspiro[4.5]-decane-2,4-dione, 3-dodecyl-1-(2,2,6,6-tetramethyl-4-piperidyl)pyrrolidin-2,5-dione, 3-dodecyl-1 -(1,2,2,6,6-pentamethyl-4-piperidyl)pyrrolidine-2,5-dione, a mixture of 4-hexadecyloxy- and 4-stearyloxy-2,2,6,6-tetramethylpiperidine, a condensation product of N,N'-bis(2,2,6,6-tetramethyl-4-piperidyl)hexamethylenediamine and 4-cyclohexylamino-2,6-dichloro-1, 3,5-triazine, N-(1,2,2,6,6-pentamethyl-4-piperidyl)-n-dodecylsuccinimide, 2-undecyl7,7,9,9-tetramethyl-1-oxa-3,8-diaza-4-oxo-spiro[4,5]decane, a reaction product of 7,7,9,9tetramethyl-2-cycloundecyl-oxa-3,8-diaza-4-oxospiro [4,5]decane and epichlorohydrin, 1,1bis (1,2,2,6,6-pentamethyl-4-piperidyloxycarbonyl)-2-(4-methoxyphenyl)ethene, N,N'-bisformyl-N,N'-bis(2,2,6,6-tetramethyl-4-piperidyl)hexamethylenediamine, diester of 4-methoxymethylene-malonic acid with 1,2,2,6,6-pentamethyl-4-hydroxy piperidine, poly(methylpropyl-3oxy-4-(2,2,6,6-tetramethyl-4-piperidyl)]-siloxane, reaction product of maleic acid anhydride-α-olefin-copolymer with 2,2,6,6-tetramethyl-4-aminopiperidine or 1,2,2,6,6-pentamethyl-4-aminopiperidine; a condensation product of 2-chloro-4,6-bis-(4-n-butylamino-1-ciclohexyloxy-2,2,6,6-tetramethylpiperidyl)-1,3,5-triazine with 1,2-bis-(3-amino-propilamino)-ethane; products known with the trade name Chinassorb 2020 and registry Number 192268-64-7or Tinuvin 123 and Registry Number 129757-67-1; products disclosed in EP 782994, US 4.477.615.
   2.7. Oxamides, such as 4,4'-dioctyloxyoxanilide, 2,2'-diethoxyoxanilide, 2,2'-dioctyloxy-5,5'-di-tert-butoxanilide, 2,2'-didodecyloxy-5,5-di-tert-butoxanilide, 2-ethoxy-2'-ethyloxanilide, N,N'-bis(3-dimethylaminopropyl)oxamide, 2-ethoxy-2'-ethyl-5,4'-di-tert-butoxanilide, mixtures of o- and p-methoxy-disubstituted oxanilides and mixtures of o- and p-ethoxy-disubstituted oxanilides.
   2.8. 2-(2-Hydroxyphenyl)-1,3,5-triazines, such as 2,4,6-tris(2-hydroxy-4-octyloxyphenyl)-1,3,5-triazine, 2-(2-hydroxy-4-octyloxyphenyl)-4,6-bis(2,4-dimethylphenyl)-1,3,5-triazine, 2,4-bis(2-hydroxy-4-propyloxyphenyl)-6-(2,4-dimethylphenyl)-1,3,5-triazine, 2-(2-hydroxy-4-octyloxyphenyl)-4,6-bis-(4-methylphenyl)-1,3,5-triazine, 2-(2-hydroxy-4-dodecyloxyphenyl)-4,6-bis(2,4-dimethylphenyl)-1,3,5-triazine, 2-(2-hydroxy-4-tridecyloxyphenyl)-4,6-bis(2,4-dimethylphenyl)-1,3,5-triazine, 2-[2-hydroxy-4-(2-hydroxy-3-butyloxy-propoxy)phenyl]-4,6-bis(2,4-dimethyl)-1,3,5-triazine, 2-[2-hydroxy-4-(2-hydroxy-3-octyloxy-propyloxy)phenyl]-4,6-bis-(2,4-dimethyl)-1,3,5-triazine, 2-[4-(dodecyloxy/tridecyloxy-2-hydroxypropoxy)-2-hydroxy-phenyl]-4,6-bis(2,4-dimethylphenyl)-1,3,5-triazine, 2-[2-hydroxy-4-(2-hydroxy-3-dodecyloxy-propoxy)phenyl]-4,6-bis(2,4-dimethylphenyl)-1,3,5-triazine, 2-(2-hydroxy-4-hexyloxy)phenyl-4,6-diphenyl-1,3,5-triazine, 2-(2-hydroxy-4-methoxyphenyl)-4,6-diphenyl-1,3,5-triazine, 2,4,6-tris[2-hydroxy-4-(3-butoxy-2-hydroxy-propoxy)phenyl]-1,3,5-triazine, 2-(2-hydroxyphenyl)-4-(4-methoxyphenyl)-6-phenyl-1,3,5-triazine, 2-(2-hydroxy-4-[3-(2-ethylhexyl-1-oxy)-2-hydroxypropyloxy]phenyl}-4,6-bis(2,4-dimethylphenyl)-1,3,5-triazine.
3. Metal deactivators, such as N,N'-diphenyloxamide, N-salicylal-N'-salicyloyl hydrazine, N,N'-bís(salicyloyl) hydrazine, N,N'-bis(3,5-di-tert-butyl-4-hydroxyphenylpropionyl)hydrazine, 3-salicyloylamino-1,2,4-triazole, bis(benzylidene)oxalyl dihydrazide, oxanilide, isophthaloyl dihydrazide, sebacoyl bisphenyilhydrazide, N,N'-diacetyladipoyldihydrazide, N,N'-bis(salicyloyl)oxalyl dihydrazide, N,N'-bis(salicyloyl)thiopropionyl dihydrazide.
4. Phosphites and phosphonites, such as triphenyl phosphite, diphenyl alkyl phosphites, phenyl dialkyl phosphites, tris(nonylphenyl) phosphite, trilauryl phosphite, trioctadecyl phosphite, distearyl pentaerythritol diphosphite, tris(2,4-di-tert-butylphenyl) phosphite, diisodecyl pentaerythritol diphosphite, bis(2,4-di-tert-butylphenyl)-phosphite; bis(2,4-di-tert-butylphenyl)-pentaerythritol diphosphite, bis-(2,6-di-tert-butyl-4-methylphenyl)-pentaerythritol-diphosphite, diisodecyloxypentaerythritol diphosphite, bis(2,4-di-tert-butyl-6-methylphenyl)pentaerythritol diphosphite, bis(2,4,6-tris(tert-butylphenyl)pentaerythritol diphosphite, trìstearyl sorbitol triphosphite,, bis(2,4-di-tert-butyl-6-methylphenyl) methyl phosphite, bis(2,4-di-tert butyl-6-methylphenyl) ethyl phosphite, 2,2',2"-nitrilo[triethyltris(3,3',5,5'-tetra-tert-butyl-1,1'-biphenyl-2,2-diyl)-phosphite], 2-ethylhexyl-(3,3',5,5'-tetra-tert-butyl-1,1'-biphenyl-2,2'-diyl)-phosphite, tetrakis(2,4-di-tert-butylphenyl) 4,4'-biphenylene diphosphonite,
5. Hydroxylamines, such as N,N-dibenzylhydroxylamine, N,N-diethylhydroxylamine, N,N-dioctylhydroxylamine, N,N-dilaurylhydroxylamine, N,N-ditetradecylhydroxylamine, N,Ndihexadecylhydroxylamine, N,N-dioctadecylhydroxylamine, N-hexadecyI-N-octadecylhydroxylamine, N-heptadecyl-N-octadecylhydroxylamine, N,N-dialkylhydroxylamine derived from hydrogenated tallow amine.
6. Nitrones, such as N-benzyl-alpha-phenyl-nitrone, N-ethyl-alpha-methyl-nitrone, N-octyl-alpha-heptyl-nitrone, N-lauryl-alpha-undecyl-nitrone, N-tetradecyl-alpha-tridecyl-nitrone, N-hexadecyl-aipha-pentadecyl-nitrone, N-octadecyl-alpha-pentadecyl-nitrone, N-heptadecyl-alpha-heptadecyl-nitrone, N-octadecyl-alpha-hexadecyl-nitrone, nitrone derived from N,N-dialkylhydroxylamine derived from hydrogenated tallow amine.
7. Thiosynergists, such as dilauryl thiodipropionate or distearyl thiodipropionate.
8. Peroxide scavengers, such as esters of β-thiodipropionic acid: for example the lauryl, stearyl, myristyl or tridecyl esters, mercaptobenzimidazole or the zinc salt of 2-mercaptobenzimidazole, zinc dibutyldithiocarbamate, dioctadecyl disulfide, pentaerythritol tetrakís(β-dodecylmercapto)propionate.
9. Polyamide stabilisers, such as copper salts in combination with iodides and/or phosphorus compounds and salts of divalent manganese.
10. Basic co-stabilisers, such as melamine, polyvinylpyrrolidone, dicyandiamide, triallyl cyanurate, urea derivatives, hydrazine derivatives, amines, polyamides, polyurethanes, alkali metal salts and alkaline earth metal salts of higher fatty acids for example calcium stearate, zinc stearate, magnesium behenate, magnesium stearate, sodium ricinoleate and potassium palmitate, antimony pyrocatecholate or zinc pyrocatecholate.
11. Nucleating agents, for example inorganic substances such as talcum; metal oxides such as titanium dioxide or magnesium oxide, phosphates, carbonates or sulfates of, preferably, alkaline earth metals; organic compounds such as mono- or polycarboxylic acids and the salts thereof, e.g. 4-tert-butylbenzoic acid, adipic acid, diphenylacetic acid, sodium succinate or sodium benzoate; polymeric compounds such as anionic copolymers.
12. Benzofuranones and indolinones, for example those disclosed in U.S. 4,325,863; U.S. 4,338,244; U.S. 5,175,312; U.S. 5,216,052; U.S. 5,252,643; DE-A-4316611; DE-A-4316622; DE-A-4316876; EP-A-0589839 or EP-A-0591102 or 3-[4-(2-acetoxyethoxy)phenyl]-5,7-di-tert-butyl-benzofuran-2-one, 5,7-di-tert-butyl-3-[4-(2-stearoyloxyethoxy)-phenyl]-benzofuran-2-one, 3,3'-bis[5,7-di-tert-butyl-3-(4-[2-hydroxyethoxy]-phenyl)-benzofuran-2-one],5,7-di-tert-butyl-3-(4-ethoxyphenyl)-benzofuran-2-one, 3-(4-acetoxy-3,5-dimethylphenyl)-5,7-di-tert-butyl-benzofuran-2-one, 3-(3,5-dimethyl-4-pivaloyloxyphenyl)-5,7-di-tert-butyl-benzofuran-2-one, 3-(2,3-di-methylphenyl)-5,7-di-tert-butyl-benzofuran-2-one; product known with the trade name Irganox HP 136 and Registry Number 181314-48-7.
13. Fillers and reinforcing agents, for example, calcium carbonate, silicates, glass fibres, asbestos, talc, kaolin, mica, barium sulfate, metal oxides and hydroxides such as calcium and zinc oxides, carbon black, graphite, wood flour and flours or fibers of other natural products, synthetic fibers.
14. Other additives, such as plasticisers, lubricants, emulsifiers, pigments, rheology additives, catalysts, flow-control agents, optical brighteners, flameproofing agents, antistatic agents and blowing agents.

The weight ratio of the compounds of the formula (I) or the mixture thereof to the conventional additives may be 1:0.5 to 1:5. Preferably, the compounds of formula (I) are mixed with UV adsorbers selected from 2-hydroxy benzophenone and 2-(2'-hydroxyphenyl)benzotriazole derivatives, benzofuranones and primary and secondary antioxidants.
The following examples illustrate the invention more specifically.

### Example 1

A mixture of 2-chloro-4,6-bis-((2,2,6,6-tetramethyl-piperidin-4-yl)butylamino)-1,3,5-triazine 134 g and 1,6-hexanediamine 500 g is warmed to 150°C and stirred for half an hour; 36 g of 30% sodium hydroxide are added slowly and the mixture warmed further to 160°C while distilling off the water from the reaction and sodium hydroxide solution. The unreacted hexanediamine is then distilled under vacuum; the residue is dissolved in 300 ml of toluene and the formed salt filtered off; the resulting solution is evaporated to dryness, at the end under vacuum. N-(2,4-bis-((2,2,6,6-Tetramethyl-piperidin-4-yl)butylamino)-1,3,5-triazin-6-il)-hexanediamine of formula XIV, melting at 45-47°C 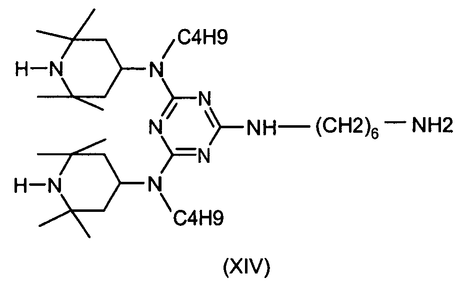 is obtained as a residue.

### Example 2

200 g of the compound of the example 1, dissolved in 400 ml of xylene, are slowly added to 29.5 g of trichlorotriazine dissolved in 280 ml of xylene. An exothermic reaction starts and the temperature rises to 60°C. At the end of the addition the mixture is stirred for ½ hour at 60°C, 36 g of 50% potassium hydroxide are added and the temperature increased to 80°C. The mixture is stirred for 1 hour and 100 ml of water are added. After the stirring is stopped, two phases separate and the aqueous phase is discarded. The organic phase is washed twice with water and the solvent evaporated warming to 150°C, at the end under high vacuum. The compound of formula (XV), melting at 98.5-100°C 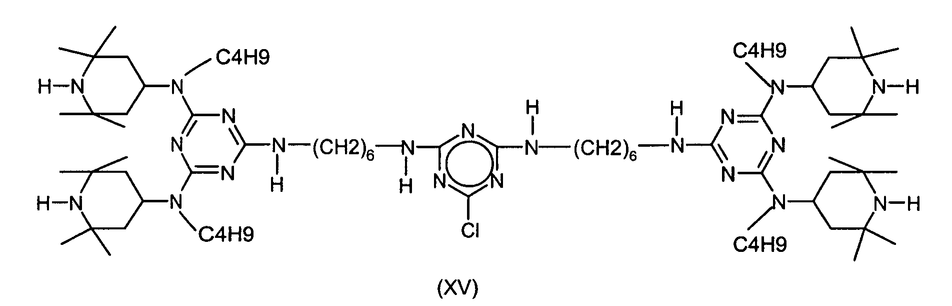 is obtained.

### Example 3

50.5 g of 2-(2,2,6,6-tetramethyl-piperidin-4-yl)butylamino-4,6-dichloro-1,3,5-triazine are dissolved in 150 ml of toluene. 8.1 g of 1,6-hexanediamine are added and the mixture is kept at 55°C under stirring for ½ hour. After adding 20.3 g of 30% sodium hydroxide, the mixture is warmed to 80°C and stirred for two hours. The obtained slurry is added with 100 ml of toluene and the water azeotropically distilled; the formed salt is filtered off and the warm solution is left to cool. The product crystallized after cooling is filtered and washed with toluene. After drying, the compound of formula XVI, melting at 144-147°C, is obtained.

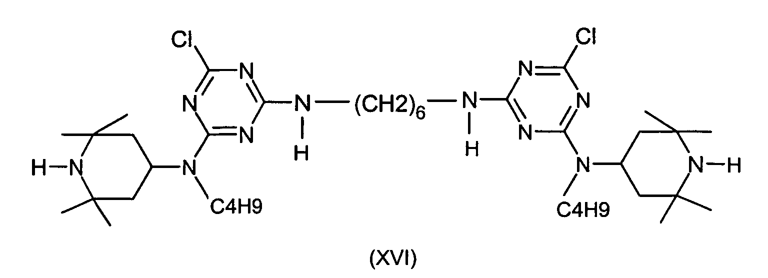

### Example 4

Following the procedure described in example 3, from 50.5 g of 2-(2,2,6,6-tetramethyl-piperidin-4-yl)butylamino-4,6-dichloro-1,3,5-triazine and 27.6 g of N,N'-bis-(2,2,6,6-tetramethyl-piperiridin-4-yl)-1,6-hexanediamine, the compound of formula XVII, melting at 107.5-109.5°C, is obtained:

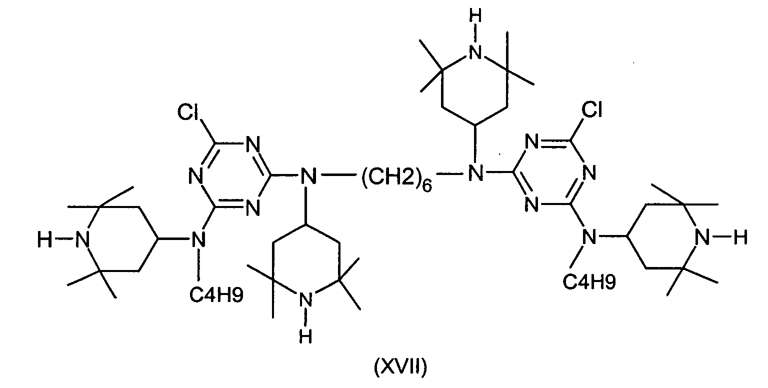

### Example 5

A mixture of 19.3 g of the compound of example 3, 32 g of the compound of example 1, 80 ml of xylene and 5.9 g of 50% potassium hydroxide is warmed at 150°C and stirred for 5 hours distilling off water. The mixture is then cooled and the salt is filtered off. The filtered solution is dried by warming to 180°C, at the end under high vacuum. The compound of general formula (I) in which:
R=H; R1=butyl; R2=R3=H; n=6
A= group of formula (II) with R=H, R1=butyl
B= group of formula (IV) with m=6; R2=R3=H
p=2
melting at 144-147°C, is obtained.

### Example 6

A mixture of 70.3 g of the compound of example 2, 2.9 g of hexanediamine, 130 ml of xylene and 6.2 g of 50% potassium hydroxide is warmed at 150°C and stirred for 5 hours distilling off the water of the mixture. 150 ml of xylene are again added, the mixture is cooled and the salt filtered off. The filtered solution is dried by warming to 150°C, at the end under high vacuum. The compound of general formula (I) in which:
R=H; R1=butyl; R2=R3=H; n=6
A= group of formula (III) in which n=6; R=H, R1= butyl; R2=R3=H
B= group of formula (IV) in which m=6; R2=R3=H
p=2
melting at 111-116°C, is obtained.

### Example 7

A mixture of 56 g of the compound of example 2, 1.4 g of diethylenetriamine, 130 ml of xylene and 5 g of 50% potassium hydroxide is warmed at 150°C and stirred for 5 hours. The procedure as described in example 5 is followed. A compound of general formula (I) in which:
R=H; R1=butyl; R2=R3=H; n=6
A= group of formula (III) in which n=6; R=H, R1= butyl; R2=R3=H
B= group of formula (V) in which r=1
p=3
melting at 130-137°C, is obtained.

### Example 8

A mixture of 56 g of the compound of example 2, 1.75 g of N,N'-(3-aminopropyl)ethylenediamine, 130 ml of xylene and 5 g of 50% KOH is warmed to 150°C and stirred for 6 hours. The procedure as described in example 5 is followed. A compound of general formula (I) melting at 125-129°C is obtained in which:
R=H; R1=butyl; R2=R3=H; n=6
A= group of formula (III) in which n=6; R=H, R1= butyl; R2=R3=H
B= group of formula (VII) in which m=2
p=4

### Example 9

Following the procedure as described in example 5, from 26 g of the compound of example 4 and 32 g of the compound of example 1, a compound of general formula (I) is obtained, melting at 124-127°C, in which:
R=H; R1=butyl; R2=R3=H; n=6
A= group of formula (II) with R1=butyl, R=H
B= group of formula (IV) in which m=6; R2=R3= group of formula (II)
p=2

### Example 10

Following the procedure as described in example 6, from 56 g of the compound of example 2 and 20.8 g of N,N'-bis-(2,2,6,6-tetramethyl-piperidin-4-yl)-1,6-hexanediamine, a compound of general formula (I) is obtained, melting at 124-130'°C, in which:
R=H; R1=butyl; R2=R3=H; n=6
A= group of formula (III), in which n=6; R=H; R1=butyl; R2=R3=H
B= group of formula (IV) in which m=6; R2=R3= group of formula (II)
p=2

### Example 11

32.7 g of the compound of example 6 are added to 250 ml of cyclohexane, followed by 0.13 g of molybdenum oxide. The solution is heated to the boiling temperature and 20.7 g of 80% tert-butylhydroperoxide are added, in about 2 hours, while azeotropically distilling off any water; during this period, 0.13 g of molybdenum oxide are added twice. The reaction mixture is then transferred, together with additional 0.3 g of molybdenum oxide, in a pressure vessel and kept at 125°C under agitation for 12 hours. After cooling, the insoluble matter is filtered off and the solution stirred with 5% sodium sulfite at 60°C for 30'; after separation of the water phase and washing with water, the organic phase is evaporated to dryness, at the end under vacuum.

A compound of general formula (I) melting at 147-154°C is obtained, in which:
R=-O-R4 with R4=cyclohexyl; R1=butyl; R2=R3=H; n=6
A= group of formula (III) in which n=6; R=-O-R4 with R4=cyclohexyl, R1=butyl; R2=R3=H
B= group of formula (IV) in which m=6; R2=R3=H
p=2

### Example 12

Following the procedure as described in example 11, from the compound of example 7 the same compound is obtained, melting at 160-168°C, in which R=-O-R4 and R4 means cyclohexyl.

### Example 13

Following the procedure as described in example 11, from the compound of example 8 the same compound is obtained, melting at 123-136°C, in which R=-O-R4 and R4 means cyclohexyl.

### Example 14

1000 g of low density polyethylene (Fertene®EF 29), 2 g of n-octadecyl-3-(3,5-di-tert-butyl-4-hydroxyphenyl)-propionate, 1 g of calcium stearate and 1.5 g of stabilizers were homogeneously blended. The mixtures were extruded at 190°C and pelletized. From the pellets, films 0.2 mm thick were obtained by pressing at 200°C. Specimens of these films were exposed to the UV radiations in a Weatherometer WOM Ci65 at a black panel temperature of 65°C. The increasing of the carbonylic absorption in the IR range at 1715 cm⁻¹ was periodically checked and the time T 0.1 required for the carbonylic band to increase to 0.1, evaluated.

The results are summarized in the Table 1.

**Table 1**

| Stabilizer | T 0.1 (hours) |
|---|---|
| Without stabilizer | 235 |
| Compound of example 5 | 4150 |
| Compound of example 6 | 4270 |
| Compound of example 8 | 4200 |
| Compound of example 10 | 4320 |
| Chimassorb ® 944 | 3850 |
| Uvasorb ® HA 88 | 4000 |

### Example 15

Homogeneous blends from polypropylene 1000 g, 1,3,5-tris-(3,5-di-tert-butyl-4-hyroxybenzyl)-cyanurate 1 g, calcium stearate 0.5 g and stabilizers 1.5 g were obtained.

The blends were extruded at 250°C and pelletized. The pellets were then spun by a fiber extruder into multifilaments fibers and tentered on a white cardboard at 120°C for 20 minutes.

Specimens of the fibers were exposed to the UV radiations in a Weatherometer WOM Ci65 at a black panel temperature of 65°C.

The photodegradation was evaluated by periodically collecting samples and measuring the time T 50 requested for reducing to halve the starting tensile strength.

The results are summarized in the Table 2.

**Table 2**

| Stabilizer | T 50 (hours) |
|---|---|
| Without stabilizer | 220 |
| Compound of example 5 | 1260 |
| Compound of example 7 | 1335 |
| Compound of example 10 | 1370 |
| Chimassorb ® 944 | 1140 |
| Uvasorb ® HA 88 | 1190 |

### Example 16

Polyethylene films were prepared according the procedure of the example 14.

Specimens of films were soaked in a 20% water solution of the pesticide Metam Sodium for 24 hours and than exposed to the UV radiations in a Weatherometer WOM in order to evaluate the time T 0.1 required to increase the carbonyl band to 0.1, as explained in example 14.

The table 3 shows the obtained results.

**Table 3**

| Stabilizer | T 0.1 (hours) |
|---|---|
| Without stabilizer | 220 |
| Compound of example 6 | 960 |
| Compound of example 8 | 890 |
| Compound of example 12 | 3920 |
| Compound of example 13 | 3870 |
| Chimassorb ® 944 | 710 |
| Uvasorb ® HA 88 | 880 |

## Claims

1. Compounds of general formula (I): 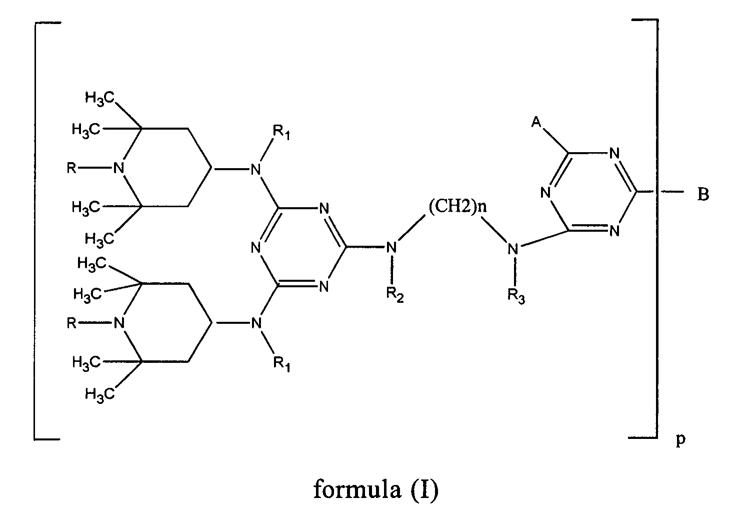 in which:
R is H, methyl or the-O-R4 group;
R4 is linear or branched C1-C8 alkyl or cyclohexyl;
R1 is hydrogen or a C1-C8 linear or branched alkyl group;
R2 and R3, which can be the same or different, are H, C1-C8 linear or branched alkyl groups or a group of formula (II) 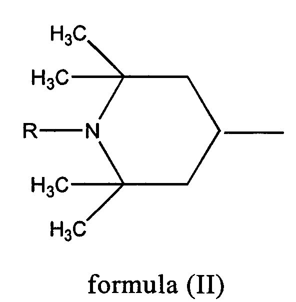
A is the-N-R2R3 group or a group of formula (III) 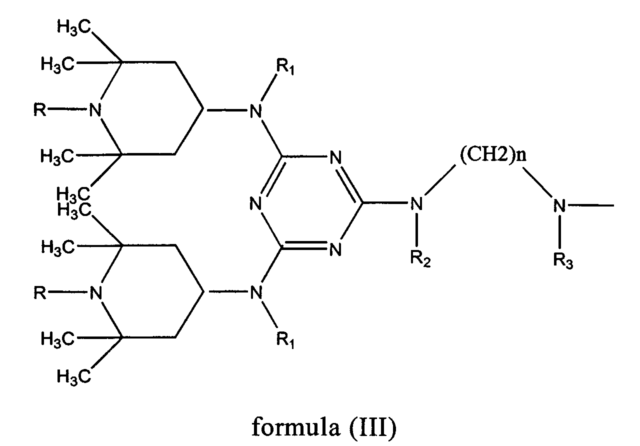
in which R, R1, R2, R3 have the meanings previously defined;
n is from 2 to 6;
p is from 2 to 5;
B means groups of formulas from (IV) to (VIII); 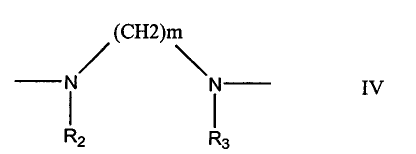 where R2 and R3 have the meanings previously defined 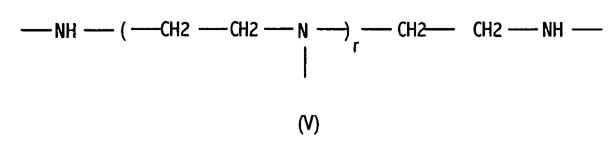 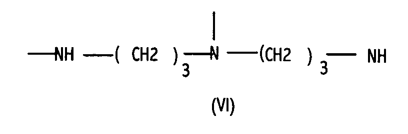 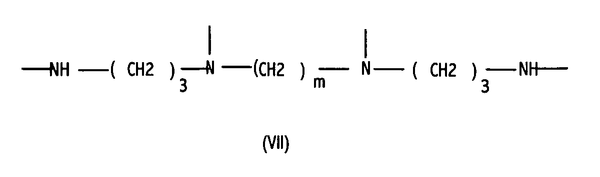 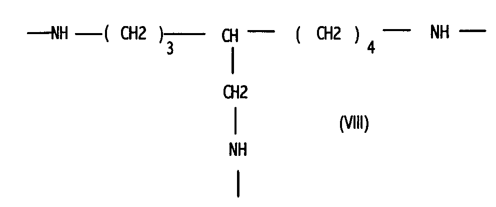
in which **m** is from 2 to 12 and **r** from 1 to 3, said compounds having well defined, not polydispersed, molecular weights.

2. Compounds according to claim 1 in which R is hydrogen or methyl.

3. Compounds according to claim 1 in which R is the-O-R4 group.

4. Compounds according to claim 3 in which R4 is cyclohexyl.

5. Compounds according to claim 1 in which R1 is hydrogen, methyl or butyl.

6. Compounds according to claim 1 in which R2 and R3, which are the same, are hydrogen or a group of formula (II).

7. Compounds according to claims 1 to 6 in which A is the group of formula (II) or the group of formula (III).

8. A process for the preparation of compounds of formula (I) as claimed in claim 1, comprising the following steps:
a) reacting a compound of formula IX: 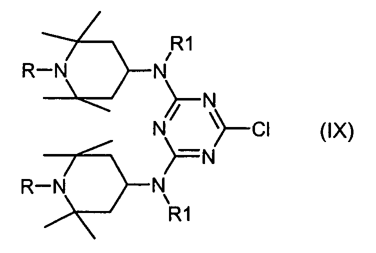 with the necessary amount of an amine of formula X: 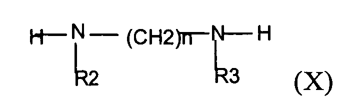 to obtain a compound of formula XI: 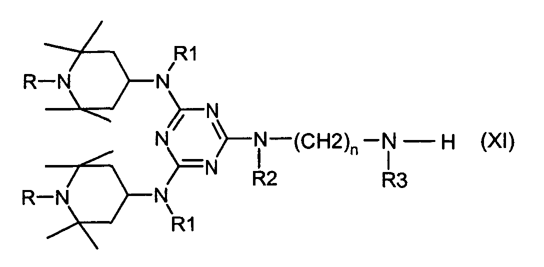
b) reacting two moles of the compound of formula XI with one mole of cyanuric chloride to obtain the intermediate of formula XII: 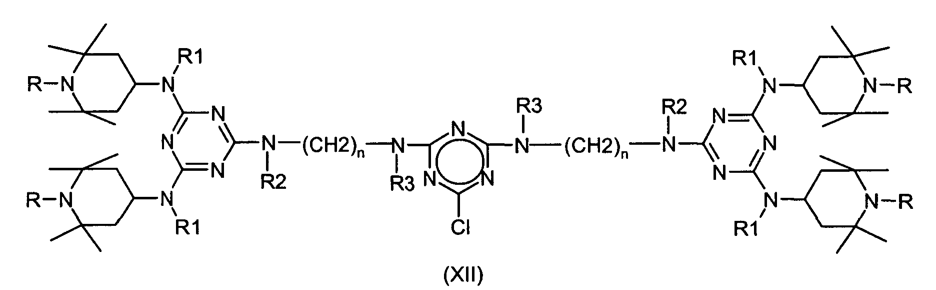
c) reacting the compound of formula XII with the polyamines containing the previous mentioned groups of formulas from IV to VIII, R, R₁, R₂ e R₄ being defined as in claim 1.

9. A process for the preparation of compounds of formula (I) which comprises reacting compounds of formula (XI) with compounds of formula (XIII) 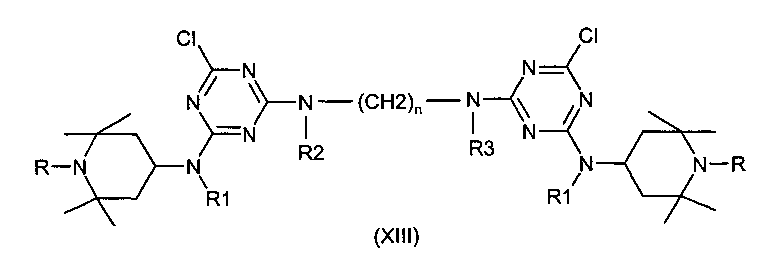 in which R, R1, R2, R3 have the meanings as defined in claim 1.

10. A process for the preparation of compounds of formula (I) in which R=-OR4 which comprises reacting the corresponding compounds of formula (I), in which R=H, with aliphatic or cycloaliphatic hydrocarbons in the presence of peroxides or hydroperoxides and molybdenum oxide.

11. A method for stabilizing organic materials with the compounds of the claims 1 to 7.

12. A method according to claim 11 in which the organic materials are polyolefins.

13. Compositions comprising an organic material sensitive to the degradation induced by light, heat and oxidation, containing at least one compound of formula (I).

14. Compositions according to claim 13 in which the organic materials are polyolefins.

15. Mixtures of the compounds of formula (I) with other Hindered Amines Light Stabilizers containing the 2,2,6,6-tetramethyl-4-piperidyl group.

16. Mixtures of the compounds of formula (I) with UV adsorbers selected form 2-hydroxy benzophenone and 2-(2'-hydroxyphenyl)benzotriazole derivatives.

17. Mixtures of the compounds of formula (I) with benzofuranones.

18. Mixtures of the compounds of formula (I) with primary and secondary antioxidants.
